# EUROPEAN PATENT SPECIFICATION

(11) **EP 2 459 555 B1**
(45) Date of publication and mention of the grant of the patent: **03.11.2021**
(21) Application number: 10740170.5
(22) Date of filing: 30.07.2010
(51) Int. Cl.: C07D 413/14, A61K 31/42, A61P 7/00

(54) **PROCESSES FOR CRYSTALLIZATION OF RIVAROXABAN**
VERFAHREN ZUR KRISTALLISIERUNG VON RIVAROXABAN
MÉTHODES DE CRISTALLISATION DE RIVAROXABAN

(30) Priority: 31.07.2009 SI 200900218
(43) Date of publication of application: 06.06.2012
(73) Proprietor: KRKA, D.D., Novo Mesto, 8501 Novo mesto (SI)
(72) Inventor: ZUPANCIC, Silvo, SI-8000 NOVO MESTO (SI); PECAVAR, Anica, SI-8000 NOVO MESTO (SI)
(74) Representative: Hoefer & Partner Patentanwälte mbB
(86) International application number: PCT/EP2010/004688
(87) International publication number: WO 2011/012321

(56) References cited:
- WO-A1-01/47919
- WO-A1-2004/060887
- WO-A1-2007/039132
- WO-A1-2009/074249
- ROEHRIG S ET AL: "Discovery of the Novel Antithrombotic Agent 5-Chloro-N-(((5S)-2-oxo-3 [4-(3-oxomorpholin-4-yl)phenyl]-1,3-oxazol idin-5-yl)methyl)thiophene 2-carboxamide (BAY 59-7939): An Oral, Direct Factor Xa Inhibitor", JOURNAL OF MEDICINAL CHEMISTRY, AMERICAN CHEMICAL SOCIETY, WASHINGTON, US LNKD- DOI:10.1021/JM050101D, vol. 48, 22 September 2005 (2005-09-22), pages 5900-5908, XP002418821, ISSN: 0022-2623 cited in the application
- None

## Description

The present invention relates to a process for preparing rivaroxaban or a pharmaceutically acceptable salt or solvate thereof.

### Background of the invention

Rivaroxaban is the INN of the anticoagulant compound 5-chloro-N-{[(5S)-2-oxo-3-[4-(3-oxomorpholin-4-yl)phenyl]oxazolidin-5-yl]methyl}thiophene-2-carboxamide, which was originally disclosed in WO 01/47919 A1. Rivaroxaban is a small molecule inhibitor of blood coagulation factor Xa and is used in the prophylaxis and treatment of thromboembolic diseases such as heart attack, angina pectoris, reocclusion and restenosis following angioplasty or bypass, cerebral apoplexy, transient ischemic attack, peripheral arterial obstructive diseases, pulmonary embolism and venous thrombosis.

Rivaroxaban corresponds to the formula (I):

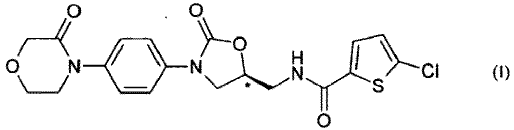

A process for the preparation of rivaroxaban, intermediates of rivaroxaban, and their preparation is disclosed for example in WO 01/47919 (Example 44). This document discloses the synthesis of rivaroxaban from an intermediate 2-({(5*S*)-2-Oxo-3-[4-(3-oxo-4-morpholinyl)phenyl]-1,3-oxazolidin-5-yl}methyl)-1*H-*isoindol-1,3(2*H*)-dion.

The process is illustrated in the following scheme:

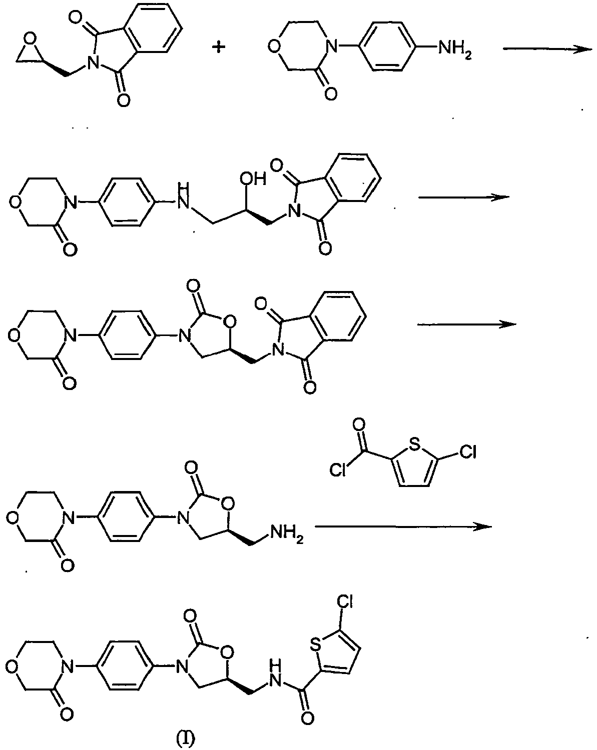

This process discloses various disadvantages in the reaction management which has particularly unfavorable effects for preparation of the rivaroxaban on the industrial scale. Furthermore, rivaroxaban is purified by "tedious chromatographic purification", i.e. by flash-chromatography from mixture dichloromethane/methanol.

Similar process is described also in Journal of Medicinal chemistry, 2005, 48, 5900-5908 and DE 10129725.

WO 2004 / 060887 discloses a method for producing rivaroxaban from 5-chlorothiophene-2-carbonyl chloride, (2S)-3-aminopropane-1,2-diol and 4-(4-aminophenyl)-3-morpholinone.

This synthesis uses toxic solvents or reagents, which is disadvantageous per se, and in addition these toxic substances must be removed from the final product for regulatory reasons, which signifies additional expense. According to description the product is obtained by precipitation and filtration after cooling the reaction mixture toluene/1-methyl-2-pyrolidone, further by washing with water and drying.

Method of purification is described also in WO2005/068456, where the crude product was suspended in acetic acid and after clarifying filtration obtained by cooling and precipitation. The precipitated product was further filtered, washed with acetic acid and water and dried.

WO2007/039132 discloses preparation of alternative forms, such as amorphous form, polymorphic form II and III. Further modifications such as hydrate, NMP solvate and inclusion compound with THF are also disclosed in the same document.

Rivoroxaban obtained according to WO 01/47919 has crystal modification which was designated as modification I and has a melting point of 232 - 233 °C.

Characteristic X-ray diffractograms of form I, II and III, hydrate and NMP solvate, are for example disclosed in WO 2007/039132. The same document discloses also IR spectrum, Raman spectrum and NIR spectrum of the same forms.

However, there are several drawbacks associated with the processes described in the art. These drawbacks include the use of tedious chromatography for purification which may not be feasible on a commercial scale, use of toxic substances during the reaction, and low yields of the product. Other drawbacks include the use of industrially less applicable solvents, such as acetic acid or NMP, therefore there is still a need for improved process of crystallization of rivaroxaban which is based on inexpensive, environmentally friendly solvents and which an be prepared using a simple and economical process that is better suited for industrial application.

Like any synthetic compound, rivaroxaban can contain extraneous compounds or impurities that can come from many sources. These can include unreacted starting materials, by-products of the reaction, products of side reactions, or degradation products. Impurities in rivaroxaban or any active pharmaceutical ingredient (API) are undesirable, and, in extreme cases, might even be harmful to a patient being treated with a dosage form of the API in which a sufficient amount of impurities are present. It is also known in the art that impurities in an API may arise from degradation of the API itself, which is related to the stability of the pure API during storage, and the manufacturing process, including the chemical synthesis.

According to CHMP ASSESSMENT REPORT FOR Xarelto (EMEA 2008), i.e. product containing rivaroxaban as an active substance, 18 process impurities originating from the starting materials and the synthetic process have been identified, including following 3 impurities: acetoxamide and bis-oxamine-urea and triamide.

Because the prior art processes do not efficiently remove certain impurities, there is also a need for improved methods of purifying rivaroxaban.

In particular, the present inventors have determined the des-chloro impurity of rivaroxaban and provided improved purification methods that reduce the level of this and other impurities in rivaroxaban.

### Description of the invention

It is an object of the present invention to provide a process for preparation rivaroxaban having desired quality properties. It is a further object of the invention to provide a process which provides crystal forms of rivaroxaban having a uniform, well-defined morphology with favorable dissolution rate, bioavailability, inter-individual variability, and chemical stability. It is another object of the present invention to provide process for rivaroxaban which exhibits advantageous physical properties, thereby improving the pharmacological properties of the drug and/or its processability when formulated to pharmaceutical dosage forms, such as tablets, capsules, pellets, granules, and powders. The rivaroxaban should have a uniform morphology meeting the above requirements and should be accessible in a reproducible manner on an industrial scale.

Still a further object of the present invention is the provision of a process for the preparation of such rivaroxaban, which can also be prepared from inexpensive, environmentally friendly solvents and can be prepared using a simple and economical process that is better suited for industrial application.

Described herein is that rivaroxaban, preferably in pure form, or a pharmaceutically acceptable salt or solvate thereof is prepared by a process which comprises the following steps:
a) dissolving rivaroxaban in a solvent or a mixture of solvents
b) crystallization of rivaroxaban
c) optionally washing and drying the crystals and
d) obtaining pure rivaroxaban.

According to one aspect of the present invention, a process for preparing rivaroxaban or a pharmaceutically acceptable salt or solvate thereof is provided which comprises the following steps:
a) dissolving rivaroxaban in a solvent or a mixture of solvents
b) crystallization of rivaroxaban
c) washing and drying the crystals and
d) obtaining pure rivaroxaban
wherein pure rivaroxaban has a purity at least 99,0 area % as determined by HPLC,
wherein the crystallization according to b) is achieved either by
(i) decreasing the temperature of a rivaroxaban crystallization mixture comprising rivaroxaban and a solvent from a temperature between room temperature and the reflux temperature of the solvent to a temperature within the range -20 °C to 40 °C,
(ii) the addition of an anti-solvent to a solution of rivaroxaban in a solvent, or
(iii) the addition of a solution of rivaroxaban in a solvent to an anti-solvent,
the solvent from step (i) being selected from 2-butanone, 3-pentanone, acetonitrile, 1-butanol and mixtures thereof, and
the solvent from steps (ii) and (iii) being selected from DMSO, DMF, DMA, acetic acid or mixtures thereof.

In the process of present invention, a pure rivaroxaban can be stable for at least 18 months when stored at 25 °C at 60 % relative humidity (RH).

In the process of present invention, the obtained product can contain less than 0,2 area % of des-chloro rivaroxaban as determined by HPLC, or
the obtained product can contain less than 0,1 area % of des-chloro rivaroxaban as determined by HPLC, or
the obtained product can contain less than 0,05 area % of des-chloro rivaroxaban as determined by HPLC.

The meaning of "pure rivaroxaban" as used herein is described further below. Preferably, the rivaroxaban dissolved in step a) above is not pure rivaroxaban, but has a lower degree of purity. Thus, a first aspect of the present invention is directed to a method of purifying or obtaining pure rivaroxaban, or a pharmaceutically acceptable salt or solvate thereof, comprising the aforementioned steps a) to d).

"Pharmaceutically acceptable salts" as used herein can preferably be salts of rivaroxaban with inorganic or organic acids, preferably salts with inorganic acids, such as, for example, hydrochloric acid, hydrobromic acid, phosphoric acid or sulphuric acid. Examples of salts with organic acids are salts with organic carboxylic or sulphonic acids, such as, for example, acetic acid, trifluoroacetic acid, propionic acid, maleic acid, fumaric acid, malic acid, citric acid, tartaric acid, lactic acid, benzoic acid, or methanesulphonic acid, ethanesulphonic acid, benzenesulphonic acid, toluenesulphonic acid or naphthalenedisulphonic acid. Other pharmaceutically acceptable salts are - without limitation - salts with customary bases, such as, for example, alkali metal salts such as sodium or potassium salts, alkaline earth metal salts such as calcium or magnesium salts or ammonium salts, derived from ammonia or organic amines, such as, for example, diethylamine, triethylamine, ethyldiisopropylamine, procaine, dibenzylamine, N-methylmorpholine, dihydroabietylamine or methylpiperidine.

The term "solvates" as used herein encompasses preferably hydrates of rivaroxaban, i.e. which form a molecule compound (solvate) in the solid or liquid state by hydration with water. In the hydrates, the water molecules are attached through secondary valencies by intermolecular forces, in particular hydrogen bridges. Solid hydrates contain water as so-called crystal water in stoichiometric ratios, where the water molecules do not have to be equivalent with respect to their binding state. Examples of hydrates are sesquihydrates, monohydrates, dihydrates or trihydrates. Equally suitable are the hydrates of salts of the compounds according to the invention. Examples of pharmaceutically acceptable salts and solvates/hydrates are also given e.g. in WO01/47919A.

According to a first variant of the process of the present invention pure rivaroxaban is achieved by providing a rivaroxaban in a solvent in order to obtain a crystallization mixture, and further by crystallizing the product from the crystallization mixture by decreasing the temperature from a first temperature T1 to a second temperature T2.

Preferred solvents for preparing the crystallization mixture are selected from polar organic solvents, more preferably they are selected from acetone, 2-butanone, 3-pentanone, acetonitrile, 1-butanol and mixtures thereof.

In order to obtain a solution the temperature of an initial suspension can be raised up to reflux temperature of the solvent. The hot crystallization mixture is then cooled. Before cooling the crystallization mixture is preferably filtered.

During the crystallization of rivaroxaban the temperature is decreased from the first temperature T1 to the second temperature T2 within the period of time 0,01 hour to 300 hours, preferably 0,1 to 50 hours, even more preferably for 1 to 5 hours. Temperature can be decreased from the temperature T1 to the temperature T2 gradually or step by step.

T1 is preferably between the room temperature (preferably 20°C) and the reflux temperature of the solvent, more preferably about the reflux temperature, and T2 is preferably within the range of -20 °C to 40 °C, more preferably 0 °C to 30 °C.

The crystals can be subjected to aging step. The crystals which form upon cooling can be kept at a temperature within the above range, i.e. preferably within the range of -20 °C to 40 °C, more preferably 0 °C to 30 °C, for 0.01 hours to 300 hours, more preferably for 0,1 to 50 hours, even more preferably for 1 to 5 hours, and are then isolated e.g. by filtration.

Optionally, the solution may be concentrated by evaporation of solvent, preferably under reduced pressure.

The crystals are preferably washed with above mentioned solvents and dried under reduced pressure of 150 to 800 mbar to obtain rivaroxaban. The drying temperatures is preferably from 30 °C to 60 °C, more preferably from 40 °C to 50 °C.

According to another variant of the process of the present invention, crystallization of rivaroxaban is induced by the addition of an anti-solvent to a solution of rivaroxaban.

A rivaroxaban solution is prepared by providing a rivaroxaban in a solvent. Temperature of the initial solution can be raised up to reflux temperature of the solvent.

Solvents according to one embodiment of the invention are preferably selected from solvents having a solubiltity of rivaroxaban about 70 g / 1 at 100 °C. They are selected from polar organic solvents. Preferred solvents are DMSO (dimethyl sulfoxide), DMF (dimethylformamide), DMA (dimethylacetamide), acetic acid or mixtures thereof.

Optionally, the solution may be concentrated by evaporation of solvent under reduced pressure.

Next, the anti-solvent is added to the solution. Preferably anti-solvent having a temperature of -20°C to 50°C, preferable 0°C to 25°C, is added to the solution of rivaroxaban that is preferably kept at a temperature between -20°C to reflux temperature, preferable between 0°C to reflux temperature. The anti-solvent is preferably added slowly, i.e. over a period of 0,01 hour to 24 hours, more preferably over a period of 0,05 hour to 10 hours, even more preferably over a period of 0,1 hour to 5 hours. During the addition of the anti-solvent the mixture is preferably stirred.

Preferred anti-solvents are selected from water, alcohols such as methanol, ethanol, isopropanol, butanol; esters such as ethyl acetate, isopropyl acetate; ethers such as THF (tetrahydrofuran), dioxane, tert-butyl methyl ether, diisopropyl ether; acetonitrile, alkanes, halogenated alkanes, aromatic carbonhydrates, and mixtures thereof.

The anti-solvent is preferably added to the solution of rivaroxaban in a volume ratio of solvent to anti-solvent from 1 : 10 to 1 : 50, preferably 1 : 15 to 1 : 25

Alternatively, the solution of the rivaroxaban can be added to the anti-solvent. In this instance, the temperature of the solution is preferably kept at temperature between -20 °C and reflux temperature, preferably between 0°C to 40°C, more preferably about at room temperature (preferably 20°C) during the addition of the anti-solvent. The anti-solvent preferably has a temperature of -20°C to 50°C, preferable -10°C to 30°C, even more preferable 0 °C to 20 °C. The solution is preferably added gradually, i.e. over a period of 0,01 hour to 24 hours, more preferably over a period of 0,05 hour to 10 hours, even more preferably over a period of 0,1 hour to 5 hours to the anti-solvent. During the addition the mixture is advantageously stirred.

Prior to mixing the solution of rivaroxaban and anti-solvent, according to one embodiment the anti-solvent can be inoculated with crystals of the desired rivaroxaban polymorphic form.

According to one embodiment, the crystals, in particular the crystals obtained in any of the processes of the invention, can be subjected to aging step. The crystals which form upon cooling can e.g. be kept at a temperature within the above range for 0.01 hours to 300 hours, more preferably for 0,1 to 50 hours, even more preferably for 1 to 5 hours, and are then isolated e.g. by filtration.

The crystals are preferably washed with above mentioned anti-solvents and preferably dried under reduced pressure of 150 to 800 mbar to obtain rivaroxaban. The drying temperature is preferably 20 °C to 60 °C, more preferably 40 °C to 50 °C.

Once crystals of rivaroxaban have been prepared, these crystals can be used as seeding material in future processes. The seeding material is preferably added during cooling of the solution in order to initiate nucleation and to shorten the aging time to obtain rivaroxaban. Seeding of the material can be applied according to any of the described variants.

"Pure rivaroxaban" means rivaroxaban having a purity at least 99,0 area %, preferably at least 99,5 area %, more preferably at least 99,7 area %. The purity can be determined using the high resolution HPLC method indicated further below.

Pure rivaroxaban according to any of the described variants can be obtained in different crystal forms, which are known from the prior art as described above. In a preferred embodiment, rivaroxaban is obtained in crystal modification form I as described in WO 2007/039132. Preferably, rivaroxaban of Form I as described in WO 2007/039132 is free of other polymorphic or amorphous forms of rivaroxaban. By "free of other polymorphic forms" it is meant that 90 to 100 % by weight, preferably at least 95 %, most preferably at least 99,8 % of the product have the desired polymorphic form.

Optically pure rivaroxaban according to the present invention is preferably used as a starting material, i.e. rivaroxaban having an optical purity within the range of 99 to 100 %, preferably higher than 99.5 % and most preferably higher than 99.8 %.

Pure rivaroxaban according to the invention is preferably stable for least 18 months stored at 25 °C / 60 % RH, and for 6 months at 40 °C /75 % RH.

The rivaroxaban of the present invention preferably has an average particle size in the range of 5 to 300 µm, preferably 20 to 150 µm, more preferably 50 to 100 µm. The term "average particle size" or "particle size" as used herein refers to the volume mean diameter of particles.

Rivaroxaban can be further micronized to obtain particles with d₉₀ < 60 µm, more preferably d₉₀ < 40 µm, and most preferably d₉₀ < 30 µm. As used herein d₉₀ < x means that at least 90 % by volume of the particles have a particle size below x.

The particle size can be determined by laser light scattering for instance using a Malvern Mastersizer Apparatus MS 2000 equipped with a Hydro S dispersion unit using purified water as the dilution medium. Micronized rivaroxaban can be obtained for instance by single or multi-stage micronization in the dry state using dry mills, such as cutting mills, pin/cage mills, hammer mills, jet mills, fluidized bed jet mills, ball mills and roller mills.

According to one embodiment, rivaroxaban can be further agglomerated or granulated. The average particle size of the agglomerated or granulated particles can e.g. be in the range of 20 to 300 µm, preferably 30 to 200 µm. The agglomeration and granulation is used to improve physical properties, such as flowability and/or compressibility of the product.

Processes of synthesis of rivaroxaban known from the prior art comprises 5-chlorothiophene-2-carbonyl chloride as starting material (Compound of formula (II), wherein R = -COCl). 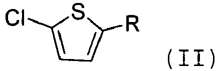 5-chlorothiophene-2-carbonyl chloride according to the prior art can be obtained from 5-chlorothiophene-2-carboxylic acid (Compound of formula (II), wherein R = -COOH).

It was surprisingly found that des-chloro compounds of tiophene ring (Compound of formula III) 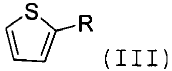 may proceed under reaction conditions to result in compound des-chloro rivaroxaban (Compound of formula IV).

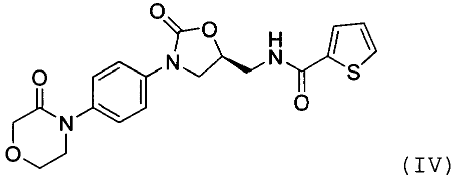

Compound of formula IV is (S)-N-((2-oxo-3-(4-(3-oxomorpholino)phenyl)oxazolidin-5-yl)methyl)thiophene-2-carboxamide, defined by chemical formula: C19H19N3O5S and molecular weight 401,44 g/mol.

Surprisingly, we found an increased purge of des-chloro compounds of formula (III) and its subsequent analogs during the reaction and isolation conditions of the synthetic process.

Compounds with formula (II) and (III) are defined with R being -COOH, COX, wherein X is halogen selected from Cl, F, Br, J, preferably X is Cl; or compounds (II) and (III) are anhydrides.

One control strategy we identified was determining the purging of the des-chloro impurities during chemical synthesis of rivaroxaban, and then setting sufficient limits on the quality of the starting reactant used to synthesize rivaroxaban. During a synthesis of rivaroxaban, one or more of the intermediates undergoes extraction and crystallization during the reaction work-ups. Although each intermediate and its corresponding des-chloro impurity are structurally similar and have similar solubility, there are slight differences in the physiochemical properties. Thus, we conducted purging experiments to determine the amount of des-chloro impurity that is removed by the extraction and recrystallization processing operations.

The process of the invention can be characterized in that the rivaroxaban is synthesized by a method of synthesizing rivaroxaban (or a pharmaceutically acceptable salt or solvate thereof) that comprises an amount of des-chloro rivaroxaban selected from not greater than about
A) 0,2 %, preferably
B) not greater than about 0,15 %, or preferably
C) not greater than about 0,10 %
which method comprises:
a) obtaining one or more samples of one or more compounds of formula (II) batches;
b) measuring the level of compounds of formula (III) in each of the samples of (a);
c) selecting a compound of formula (II) batch that comprises a purity of compound of formula (II) based on the measurement of compound of formula (III) conducted in (b); and
d) using the batch selected in (c) to synthesize rivaroxaban.

Purity of the compound of formula (II) can preferably be greater than about 97 %, preferably greater than about 98 %, most preferably greater than about 99 %. Preferably, the level of compounds of formula (III) in the compound of formula (II) batch used is less than about 3 %, preferably less than about 2 %, more preferably less than about 1 %, more preferably less than about 0.5 %, more preferably less than about 0.1 %. The purity/level can be determined as area % for instance using HPLC method as known in the prior art.

Rivaroxaban according to the present invention is therapeutically stable and is particularly suitable for preparing medicaments.

The pharmaceutical compositions may be in a solid or liquid dosage form. Exemplary solid dosage forms include tablets, capsules, sachets, lozenges, powders, pills, pellets, or granules. The solid dosage form may be, for example, a immediate release dosage form, a fast melt dosage form, orally disintegrating dosage form, modified release dosage form, lyophilized dosage form, delayed release dosage form, extended release dosage form, prolonged release dosage form, pulsatile dosage form, mixed immediate and modified release dosage form, or a combination thereof. Solid dosage forms are preferred. More preferably, the solid dosage form is an immediate release dosage form offering advantages regarding the bioavailability of the active compound.

Pharmaceutical dosage forms comprising rivaroxaban can be prepared by a process comprising the steps of mixing rivaroxaban according to the present invention with at least one pharmaceutically acceptable excipient and forming the mixture into a pharmaceutical dosage form. Rivaroxaban and the one or more excipients can be mixed in the presence or in the absence of solvent.

Aspects of the invention are directed to the following items:
1. Process for preparing rivaroxaban, preferably pure rivaroxaban, or a pharmaceutically acceptable salt or solvate thereof comprising the following steps:
   a) dissolving rivaroxaban in a solvent or a mixture of solvents
   b) crystallization of rivaroxaban
   c) washing and drying the crystals and
   d) obtaining pure rivaroxaban
   wherein pure rivaroxaban has a purity at least 99,0 area % as determined by HPLC,
   wherein the crystallization according to b) is achieved either by
   (i) decreasing the temperature of a rivaroxaban crystallization mixture from a temperature between room temperature and the reflux temperature of the solvent to a temperature within the range -20 °C to 40 °C,
   (ii) the addition of an anti-solvent to a solution of rivaroxaban in a solvent, or
   (iii) the addition of a solution of rivaroxaban in a solvent to an anti-solvent,
   the solvent from step (i) being selected from 2-butanone, 3-pentanone, acetonitrile, 1-butanol and mixtures thereof, and
   the solvent from steps (ii) and (iii) being selected from DMSO, DMF, DMA, acetic acid or mixtures thereof.
2. The process according to item 1, characterized in that the anti-solvent from steps (ii) and (iii) is selected from water, alcohols such as methanol, ethanol, isopropanol, butanol; esters such as ethyl acetate, isopropyl acetate; ethers such as THF, dioxane, tert-butyl methyl ether, diisopropyl ether; acetonitrile, alkanes, halogenated alkanes, aromatic carbonhydrates, and mixtures thereof.
3. The process of any of items 1 to 2, characterized in that the pure rivaroxaban has purity at least 99,5 area % as determined by HPLC.
4. The process of any of items 1 to 3, characterized in that pure rivaroxaban has purity at least 99,7 area % as determined by HPLC.
5. The process of any of the preceding items wherein a pure rivaroxaban is in a crystal form I.
6. The process of any of the preceding items wherein a pure rivaroxaban is stable for at least 18 months when stored at 25 °C at 60 % relative humidity (RH).
7. The process of any of the preceding items wherein obtained product contains less than 0,2 area % of des-chloro rivaroxaban as determined by HPLC.
8. The process of any of the preceding items wherein obtained product contains less than 0,1 area % of des-chloro rivaroxaban as determined by HPLC.
9. The process of any of the preceding items wherein obtained product contains less than 0,05 area % of des-chloro rivaroxaban as determined by HPLC.
10. The process according to any of the preceding items characterized in that the rivaroxaban is synthesized by a method of synthesizing a rivaroxaban that comprises an amount of des-chloro rivaroxaban selected from not greater than about
   A) 0,2 %,
   B) not greater than about 0,15 %, or
   C) not greater than about 0,10 %
   which method comprises:
   a) obtaining one or more samples of one or more compounds of formula (II) batches; 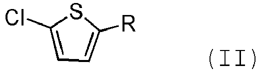
   b) measuring the level of compounds (III) 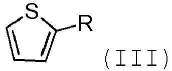 in each of the samples of (a);
   c) selecting a compound of formula (II) batch that comprises a purity of compound of formula (II) based on the measurement of compound of formula (III) conducted in (b); and
   d) using the batch selected in (c) to synthesize rivaroxaban,
   wherein R of formulas (II) and (III) are defined with R being - COOH, COX, wherein X is halogen selected from Cl, F, Br, J, or compounds (II) and (III) are anhydrides.

Described herein is a pharmaceutical composition comprising pure rivaroxaban obtained according to any of the preceding items.

According to one embodiment, the % values regarding the amount of des-chloro rivaroxaban and the % purity values regarding rivaroxaban as given herein are area % values, in particular as determined by the high resolution HPLC method described herein.

In the following the invention will be described by use of examples. In the examples, high resolution HPLC is used to determine the purity of rivaroxaban. The tests are carried out in Hypersil BDS C18 (150 x 4.6 mm i.d, 3 µm particles) column The mobile phase is a gradient of eluents A and B. (Eluent A:0.01M ammonium dihydrogen phosphate, pH adjusted to 5.0 with phosphoric acid; Eluent B: acetonitrile : water) The chromatograph is equipped with a UV detector set at 250 nm, flow rate is 1.0 ml/min at 25°C. Results are expressed as area %.

The X-ray powder diffraction patterns were obtained on a Philips PW3040/60 X' Pert powder diffractometer, X' celerator detector at CuKa radiation, 1.54178 A, 3°<20<30°.

### Examples:

### Example 1:

The mixture of 1g rivaroxaban and 2 ml of dimethylsulfoxide was heated to obtain clear solution. The solution was slowly cooled and during this process the product was precipitated. To the obtained suspension cooled to room temperature 10 ml tetrahydrofurane was gradually added. The suspension was stirred at room temperature for 0.5 hour and 2 hours at 0°C. The precipitate was filtered washed with water and dried at 40 to 50°C under reduced pressure.

### Yield: 71%

HPLC purity: 99.74 %, Imp. (Rt 7.0 min): 0.06 %, Des-chloro rivaroxaban impurity (Rt 13.7 min) : 0.07 %

### Example 2:

The mixture of 1g rivaroxaban and 5 ml of dimethylsulfoxide was heated to obtain clear solution. The hot solution was slowly poured into 20 ml of water. The obtained suspension was stirred at room temperature for 2 hours. The precipitate was filtered washed with water and dried at 40 to 50°C under reduced pressure.

### Yield: 94 %

HPLC purity: 99.44 %, Imp. (Rt 7.0 min): 0.03 %, Des-chloro rivaroxaban 0.11 %

### Example 3:

The mixture of 1g rivaroxaban and 2 ml of dimethylsulfoxide was heated to obtain clear solution. The solution was slowly cooled and during this process the product was precipitated. To the obtained suspension cooled to room temperature 10 ml acetonitrile was gradually added. The suspension was stirred at room temperature for 0.5 hour and 2 hours at 0°C. The precipitate was filtered washed with water and dried at 40 to 50°C under reduced pressure.

### Yield: 84 %

HPLC purity: 99.70 %, Imp. (Rt 7.0 min): 0.06 %, Des-chloro rivaroxaban 0.08 %

### Example 4:

The mixture of 1g rivaroxaban and 5 ml of dimethylsulfoxide was heated to obtain clear solution. The solution was slowly cooled and during this process 7 ml ethanol was gradually added. The product was precipitated and the suspension was stirred at room temperature for 0.5 hour and 2 hours at 0°C. The precipitate was filtered washed with ethanol and dried at 40 to 50°C under reduced pressure.

### Yield: 93%

HPLC purity: 99.52 %, Imp. (Rt 7.0 min): 0.10 %, Des-chloro rivaroxaban 0.09 %

### Example 5:

The mixture of 1g rivaroxaban and 2 ml of dimethylsulfoxide was heated to obtain clear solution. The solution was slowly cooled and during this process the product was precipitated. To the obtained suspension cooled to room temperature 10 ml ethyl acetate was gradually added. The suspension was stirred at room temperature for 0.5 hour and 2 hours at 0°C. The precipitate was filtered washed with water and dried at 40 to 50°C under reduced pressure.

### Yield: 93 %

HPLC purity: 99.48 %, Imp. (Rt 7.0 min): 0.11 %, Des-chloro rivaroxaban 0.09 %

### Example 6:

The mixture of 1g rivaroxaban and 2 ml of dimethylsulfoxide was heated to obtain clear solution. The solution was slowly cooled and during this process the product was precipitated. To the obtained suspension cooled to room temperature 10 ml water was gradually added. The suspension was stirred at room temperature for 0.5 hour and 2 hours at 0°C. The precipitate was filtered washed with water and dried at 40 to 50°C under reduced pressure.

### Yield: 97 %

HPLC purity: 99.25 %, Imp. (Rt 7.0 min): 0.20 %, Des-chloro rivaroxaban 0.12 %

### Example 7:

The mixture of 1g rivaroxaban and 5 ml of N,N-dimethyformamide was heated to obtain clear solution. The solution was slowly cooled and during this process the product was precipitated. To the obtained suspension cooled to room temperature 25 ml of acetone was gradually added. The suspension was stirred at room temperature for 0.5 hour and 2 hours at 0°C. The precipitate was filtered washed with water and dried at 40 to 50°C under reduced pressure.

### Yield: 86 %

HPLC purity: 99.56 %, Imp. (Rt 7.0 min): 0.08 %, Des-chloro rivaroxaban 0.09 %

### Example 8:

The mixture of 1g rivaroxaban and 5 ml of N,N-dimethyformamide was heated to obtain clear solution. The hot solution was slowly poured into 25 ml of acetone. The obtained suspension was stirred at room temperature for 2 hours. The precipitate was filtered washed with water and dried at 40 to 50°C under reduced pressure.

### Yield: 90%

HPLC purity: 99.51 %, Imp. (Rt 7.0 min): 0.04 %, Des-chloro rivaroxaban 0.11 %

### Example 9:

The mixture of 1g rivaroxaban and 10 ml of *N,N-*dimethyformamide was heated to obtain clear solution. The solution was slowly cooled and during this process the product was precipitated. To the obtained suspension cooled to room temperature 20 ml of acetone was gradually added. The suspension was stirred at room temperature for 0.5 hour and 2 hours at 0°C. The precipitate was filtered washed with water and dried at 40 to 50°C under reduced pressure.

### Yield: 92 %

HPLC purity: 99.38 %, Imp. (Rt 7.0 min): 0.15 %, Des-chloro rivaroxaban 0.10 %

### Example 10:

The mixture of 1g rivaroxaban and 5 ml of N,N-dimethyformamide was heated to obtain clear solution. The solution was slowly cooled and during this process the product was precipitated. To the obtained suspension cooled to room temperature 25 ml of tetrahydrofurane was gradually added. The suspension was stirred at room temperature for 0.5 hour and 2 hours at 0°C. The precipitate was filtered washed with water and dried at 40 to 50°C under reduced pressure.

### Yield: 71 %

HPLC purity: 99.58 %, Imp. (Rt 7.0 min): 0.08 %, Des-chloro rivaroxaban 0.09 %

### Example 11:

The mixture of 1g rivaroxaban and 10 ml of *N,N-*dimethyformamide was heated to obtain clear solution. The solution was concentrated to obtain solid residue which is suspended in 20 ml of ethanol. The suspension was stirred at room temperature for 2 hours. The precipitate was filtered washed with ethanol and dried at 40 to 50°C under reduced pressure.

### Yield: 96 %

HPLC purity: 99.26 %, Imp. (Rt 7.0 min): 0.21%, Des-chloro rivaroxaban 0.11 %

### Example 12:

The mixture of 1g rivaroxaban and 10 ml of *N,N-*dimethyformamide was heated to obtain clear solution. The solution was concentrated to obtain solid residue which is suspended in 20 ml of ethyl acetate. The suspension was stirred at room temperature for 2 hours. The precipitate was filtered washed with ethyl acetate and dried at 40 to 50°C under reduced pressure.

### Yield: 97 %

HPLC purity: 99.36 %, Imp. (Rt 7.0 min): 0.16 %, Des-chloro rivaroxaban 0.11 %

### Example 13:

The mixture of 1g rivaroxaban and 10 ml of *N,N-*dimethyformamide was heated to obtain clear solution. The solution was concentrated to obtain solid residue which is suspended in 20 ml of tetrahydrofurane. The suspension was stirred at room temperature for 2 hours. The precipitate was filtered washed with tetrahydrofurane and dried at 40 to 50°C under reduced pressure.

### Yield: 96 %

HPLC purity: 99.33 %, Imp. (Rt 7.0 min): 0.17 %, Des-chloro rivaroxaban 0.11 %

### Example 14:

The mixture of 1g rivaroxaban and 10 ml of *N,N-*dimethyformamide was heated to obtain clear solution. The solution was concentrated to obtain solid residue which is suspended in 20 ml of isopropyl acetate. The suspension was stirred at room temperature for 2 hours. The precipitate was filtered washed with isopropyl acetate and dried at 40 to 50°C under reduced pressure.

### Yield: 99 %

HPLC purity: 99.28 %, Imp. (Rt 7.0 min): 0.22 %, Des-chloro rivaroxaban 0.11 %

### Example 15:

The mixture of 1g rivaroxaban and 10 ml of *N,N-*dimethyformamide was heated to obtain clear solution. The solution was concentrated to obtain solid residue which is suspended in 20 ml of water. The suspension was stirred at room temperature for 2 hours. The precipitate was filtered washed with water and dried at 40 to 50°C under reduced pressure.

### Yield: 98 %

HPLC purity: 99.23 %, Imp. (Rt 7.0 min): 0.19 %, Des-chloro rivaroxaban 0.11 %

### Example 16:

The mixture of 1g rivaroxaban and 5 ml of N,N-dimethyformamide was heated to obtain clear solution. The solution was slowly cooled and during this process the product was precipitated. To the obtained suspension cooled to room temperature 25 ml of water was gradually added. The suspension was stirred at room temperature for 0.5 hour and 2 hours at 0°C. The precipitate was filtered washed with water and dried at 40 to 50°C under reduced pressure.

### Yield: 97%

HPLC purity: 99.27 %, Imp. (Rt 7.0 min): 0.21 %, Des-chloro rivaroxaban 0.11 %

### Example 17:

The mixture of 1g rivaroxaban and 5 ml of N,N-dimethyacetamide was heated to obtain clear solution. The solution was slowly cooled and during this process the product was precipitated. To the obtained suspension cooled to room temperature 20 ml of water was gradually added. The suspension was stirred at room temperature for 0.5 hour and 2 hours at 0°C. The precipitate was filtered, washed with water and dried at 40 to 50°C under reduced pressure.

### Yield: 86%

HPLC purity: 99.14 %, Imp. (Rt 7.0 min): 0.03 %, Des-chloro rivaroxaban 0.11 %

### Example 18:

The mixture of 1g rivaroxaban and 10 ml of *N,N-*dimethyacetamide was heated to obtain clear solution. The solution was slowly cooled and during this process the product was precipitated. To the obtained suspension cooled to room temperature 4 ml of water was gradually added. The suspension was stirred at room temperature for 0.5 hour and 2 hours at 0°C. The precipitate was filtered washed with water. To the wet product 10 ml of ethanol was added and the mixture was stirred at room temperature for 1.5 hour. The product was filtered, washed with ethanol and dried at 40 to 50°C under reduced pressure.

### Yield: 93%

HPLC purity: 99.52 %, Imp. (Rt 7.0 min): 0.08 %, Des-chloro rivaroxaban 0.10 %

### Example 19:

The mixture of 1g rivaroxaban and 10 ml of *N,N-*dimethyacetamide was heated to obtain clear solution. The solution was slowly cooled and during this process the product was precipitated. To the obtained suspension cooled to room temperature 50 ml of acetone was gradually added. The suspension was stirred at room temperature for 0.5 hour and 2 hours at 0°C. The precipitate was filtered, washed with water and dried at 40 to 50°C under reduced pressure.

### Yield: 82%

HPLC purity: 99.61 %, Imp. (Rt 7.0 min): 0.09 %, Des-chloro rivaroxaban 0.08 %

### Example 20:

The mixture of 1g rivaroxaban and 20 ml of acetic acid was heated to obtain clear solution. The solution was slowly cooled and during this process the product was precipitated. To the obtained suspension cooled to room temperature 40 ml of acetone was gradually added. The suspension was stirred at room temperature for 0.5 hour and 2 hours at 0°C. The precipitate was filtered, washed with acetone and dried at 40 to 50°C under reduced pressure.

### Yield: 80%

HPLC purity: 99.62 %, Imp. (Rt 7.0 min): 0.07 %, Des-chloro rivaroxaban 0.09 %

### Example 21:

The mixture of 1g rivaroxaban and 20 ml of acetic acid was heated to obtain clear solution. The solution was slowly cooled and during this process the product was precipitated. To the obtained suspension cooled to room temperature 40 ml of TBME was gradually added. The suspension was stirred at room temperature for 0.5 hour and 2 hours at 0°C. The precipitate was filtered, washed with TBME and dried at 40 to 50°C under reduced pressure.

### Yield: 96 %

HPLC purity: 99.51 %, Imp. (Rt 7.0 min): 0.07 %, Des-chloro rivaroxaban 0.11 %

### Example 22:

The mixture of 1g rivaroxaban and 20 ml of acetic acid was heated to obtain clear solution. The solution was slowly cooled and during this process the product was precipitated. The suspension was stirred at room temperature for 0.5 hour and 2 hours at 0°C. The precipitate was filtered, washed with water and dried at 40 to 50°C under reduced pressure.

### Yield: 86 %

HPLC purity: 99.54 %, Imp. (Rt 7.0 min): 0.06 %, Des-chloro rivaroxaban 0.11 %

### Example 23:

The mixture of 1g rivaroxaban and 20 ml of acetic acid was heated to obtain clear solution. The hot solution was slowly poured into 30 ml of ethanol. The obtained suspension was stirred at room temperature for 3 hours. The precipitate was filtered, washed with ethanol and dried at 40 to 50°C under reduced pressure.

### Yield: 89 %

HPLC purity: 99.49 %, Im. (Rt 7.0 min): 0.09 %, Des-chloro rivaroxaban 0.10 %

### Example 24:

The mixture of 1g rivaroxaban and 20 ml of acetic acid was heated to obtain clear solution. The hot solution was slowly poured into 30 ml of isopropyl acetate. The obtained suspension was stirred at room temperature for 3 hours. The precipitate was filtered, washed with isopropyl acetate and dried at 40 to 50°C under reduced pressure.

### Yield: 91 %

HPLC purity: 99.43 %, Imp. (Rt 7.0 min): 0.11 %, Des-chloro rivaroxaban 0.10 %

### Example 25:

The mixture of 1g rivaroxaban and 20 ml of acetic acid was heated to obtain clear solution. The hot solution was slowly poured into 30 ml of 1-butanol. The obtained suspension was stirred at room temperature for 3 hours. The precipitate was filtered, washed with 1-butanol and dried at 40 to 50°C under reduced pressure.

### Yield: 90 %

HPLC purity: 99.42 %, Imp. (Rt 7.0 min): 0.09 %, Des-chloro rivaroxaban 0.11 %

### Example 26:

The mixture of 1g rivaroxaban and 20 ml of acetic acid was heated to obtain clear solution. The hot solution was slowly poured into 30 ml of water. The obtained suspension was stirred at room temperature for 3 hours. The precipitate was filtered, washed with water and dried at 40 to 50°C under reduced pressure.

### Yield: 92 %

HPLC purity: 99.62 %, Imp. (Rt 7.0 min): 0.06 %, Des-chloro rivaroxaban 0.09 %

### Example 27:

The mixture of 1g rivaroxaban and 20 ml of acetic acid was heated to obtain clear solution. The hot solution was slowly poured into 30 ml of 1,4-dioxane. The obtained suspension was stirred at room temperature for 3 hours. The precipitate was filtered, washed with 1,4-dioxane and dried at 40 to 50°C under reduced pressure.

### Yield: 81%

HPLC purity: 99.57 %, Imp. (Rt 7.0 min): 0.07 %, Des-chloro rivaroxaban 0.09 %

### Example 28:

The mixture of 1g rivaroxaban and 52 ml of acetonitrile was heated to obtain clear solution. The hot solution was filtered, cooled to room temperature and stirred at room temperature for 2 hours and at 0°C for 0.5 hour. The precipitate was filtered, washed with acetonitrile and dried at 40 to 50°C under reduced pressure.

### Yield: 97 %

HPLC purity: 99.67 %, Imp. (Rt 7.0 min): 0.03 %, Des-chloro rivaroxaban 0.08 %

### Example 29 (for comparison):

The mixture of 1g rivaroxaban and 30 ml of acetone was heated to obtain clear solution. The hot solution was filtered, cooled to room temperature and stirred at room temperature for 2 hours and at 0°C for 0.5 hour. The precipitate was filtered, washed with acetone and dried at 40 to 50°C under reduced pressure.

### Yield: 94 %

HPLC purity: 99.50 %, Imp. (Rt 7.0 min): 0.13 %, Des-chloro rivaroxaban 0.09 %

### Example 30:

The mixture of 1g rivaroxaban and 30 ml of 2-butanone was heated to obtain clear solution. The hot solution was filtered, cooled to room temperature and stirred at room temperature for 2 hours and at 0°C for 0.5 hour. The precipitate was filtered, washed with 2-butanone and dried at 40 to 50°C under reduced pressure.

### Yield: 93 %

HPLC purity: 99.52 %, Imp. (Rt 7.0 min): 0.11 %, Des-chloro rivaroxaban 0.09 %

### Example 31:

The mixture of 1g rivaroxaban and 30 ml of 1-butanol was heated to obtain clear solution. The hot solution was filtered, cooled to room temperature and stirred at room temperature for 2 hours and at 0°C for 0.5 hour. The precipitate was filtered, washed with 1-butanol and dried at 40 to 50°C under reduced pressure.

### Yield: 97 %

HPLC purity: 99.50 %, Imp. (Rt 7.0 min) : 0.09 %, Des-chloro rivaroxaban 0.10 %

Rivaroxaban according to examples 1 to 31 was obtained in crystal form I.

### Example 32: Synthesis of rivaroxaban

### a.) Synthesis of 4-phenylmorpholin-3-one

In a 5L four-necked flask, water (3L), ethanol (1L) and 2-(phenylamino)ethanol (1kg, 7.300mol) were heated to 38 °C while stirring. Chloroacetyl chloride (2.47kg, 3eq) and 45% sodium hydroxide solution (NaOH 1.9kg/H₂O 3.1 1, 6.2eq) were then added simultaneously at temperature of 38-45 °C within 60 to 80 minutes; pH 10 to 13; and stirred at this temperature for 30 minutes. It was further cooled to 8 °C and stirred at this temperature for another 30 minutes. The precipitated product was filtered off and washed twice with 2.5kg water, each time of demineralised water at 2 °C. The moist product is dried to constant mass at 50 °C under reduced pressure to obtain 1.4kg of product (Yield 68%).

### b.) Synthesis of 4-(4-nitrophenyl)-3-morpholinone

In a three-necked flask a compound obtain according to a.) (500 g, 2.824 mol) was introduced at temperature 10 °C in 4 portions into of concentrated sulphuric acid (1L) within 30minutes . The mixture was then heated to 30 °C for 30 minutes. The solution is cooled to -5 °C and admixed within one hour with 65% nitric acid (205ml, 0.98 eq). The mixture was stirred at -5 °C for one hour. Then the mixture was put into ice-water (2.5kg), the precipitate was filtered and washed by water (0.5L*2). 1300 mL of water are metered into the filter at 10 °C, with 25% aqueous ammonia solution (200mL). The suspension is admixed with 7L of acetone and heated to 50 °C. Furtheron, the phases were be separated; 12L of acetone/water mixture were distilled of which the product precipitates out. The suspension was cooled to 10 °C and stirred for a further 30 minutes, and the product was isolated. The moist product wass washed with 400mL of cold acetone and dried at 50 °C under reduced pressure to obtain a 432 g of the product (Yield 69 %).

### c.) Synthesis of 4-(4-aminophenyl)morpholin-3-one

In a 5L flask, MeOH (2L), 4-(4-nitrophenyl)-3-morpholinone (204g), Raney Ni(50g), was stirred at 40∼50 °C under the atmosphere of H₂ for 24hours, and the catalyst was filtered off. The solution was concentrated under reduced pressure and remaining solid was dried to constant weight at 50 °C under reduced pressure. An amount of 148.3g of the product was obtained (Yield 85%).

### d.) Synthesis of (R)-2-(2-hydroxy-3-(4-(3-oxomorpholino) phenylamino) propyl) isoindoline-1,3-dione

A suspension of (S)-2-(oxiran-2-ylmethyl)isoindoline-1,3-dione (4.5kg, 1.22eq) and 4-(4-aminophenyl)morpholin-3-one (3.286kg, 17.088 mol) in ethanol/water (42L/16kg), was heated at 60 °C for 36 hours. It was further cooled to room temperature, the precipitate was filtered, and dried in vacuum. An amount of 5.1 kg of the product was obtained (Yield 75,4%).

### e.) Synthesis of (S)-2-((2-oxo-3-(4-(3-oxomorpholino) phenyl) oxazolidin-5-yl) methyl) isoindoline-1,3-dione

N,N-Carbonyldiimidazole (2.897kg, 1.47eq) was added to a suspension of compound according to d.) (5.1kg, 12.114mol)in toluene(49L). The reaction mixture was refluxed for 3h, then at 60 °C, ethanol (14L) was added. After cooling to 25 °C,the precipitate was filtered and 4.8 kg of (S)-2-((2-oxo-3-(4-(3-oxomorpholino)phenyl) oxazolidin-5-yl) methyl) isoindoline-1,3-dione was obtained as a colorless solid (Yield 88,3 %).

### f.) Synthesis of (S)-4-(4-(5-(aminomethyl) -2-oxooxazolidin-3-yl) phenyl) morpholin-3-one

Methylamine (35% in ethanol, 5kg) was added to a suspension of compound according to e.) (4.79kg) in ethanol (36kg). The reaction mixture was heated to 65 °C for 4 hours. After cooling to 58-60 °C, a total of 7L of 5M hydrochloric acid solution is added until the pH is 2.7, after which the product starts to crystallize. After cooling to 20 °C, the precipitated reaction product is filtered off with suction, washed with CH₂Cl₂ (8L*2) and then dried to obtain 3.11kg of the product (Yield 83.7%).

### g.) Synthesis of rivaroxaban

N,N-Carbonyldiimidazole (1.444 kg,1.2eq) was added slowly to a suspension of 5-chlorothiophene-2-carboxylic acid (1200g, 7.430mol) in DMF(8L) below 0 °C, and stirred for 1 hour at room temperature, then Et₃N (900g, 1.2eq) was added to the mixture at 0 °C , and compound according to f.) (2500g, 1.03eq) was further added within 30 minutes and stirred for 2hours at 25 °C. Ice-water (10kg) was added and the product then precipitated from the solution. The product was filtered off and the crude product (2400g) has been obtained.

### h.) Crystallization

2400g of crude product according to g.) was suspended in 8L of acetic acid and heated to 110-115 °C. The resulting solution was stirred at this temperature for 15 minutes, after clarifying filtration, cooled to 20 °C. The precipitated product was filtered off with suction, washed with acetic acid and water and then dried. An amount of 2.020 kg of rivaroxaban was obtained.

### Example 33:

### Single-stage micronization of rivaroxaban

Rivaroxaban was micronized using single-stage milling. A fluid jet mill with Venturi inlet nozzle operating at a pressure of about 12 bar, a ring pressure of about 11 bar and a dosing mass flow of 20-30 g/min was used (Jetmill MC50, Jet Pharma S.A.). The resultant rivaroxaban was determined to have a particle size distribution of d₉₀ < 15 µm.

The specific surface area of the rivaroxaban was measured by a gas sorption system based on the nitrogen adsorption, using the 6-point Brunauer, Emmett and Teller (BET) method. The measurement showed a change from 0.4 m²/g before milling to 7 m²/g after micronization.

### Example 34

### Multi-stage micronization of rivaroxaban

Rivaroxaban was micronized using multi-stage milling. A fluid jet mill with Venturi inlet nozzle operating at a pressure of about 6 bar, a ring pressure of about 4 bar and a dosing mass flow of 20-30 g/min was used in the first stage (Jetmill MC50, Jet Pharma S.A.). The resultant rivaroxaban was determined to have a particle size distribution of d₉₀ < 50 µm.

Subsequently, the rivaroxaban was micronised in a second stage with the Venturi inlet nozzle operating at a pressure of about 12 bar, a ring pressure of about 11 bar and a dosing mass flow of 20-30 g/min (Jetmill MC50, Jet Pharma S.A.). The resultant rivaroxaban was determined to have a particle size distribution of d₉₀ < 10 µm.

### Example 35 Co-milling of rivaroxaban together with hydrophilic excipient

50 g of rivaroxaban and 100 g of sorbitol were co-milled using an air-jet mill with an inlet air pressure of about 9 bar and a milling pressure of about 8.5 bar. The resultant mixture of co-milled rivaroxaban and hydrophilic excipient was micronized as described above in example 33 or 34.

The following pharmaceutical compositions were prepared with rivaroxaban according to invention.

### Example 36

### Preparation of tablets by direct compression

**Tablet compositions were prepared as follows:**

| | **4A** | **4B** | **4C** | **4D** | **4E** | **4F** | **4G** |
|---|---|---|---|---|---|---|---|
| Rivaroxaban | 10.0 | 10.0 | 10.0 | 10.0 | 10.0 | 10.0 | 10.0 |
| Sodium lauryl sulfate | 2.0 | 2.0 | 2.0 | 2.0 | 2.0 | 2.5 | 2.5 |
| Mannitol | 42.5 | 42.5 | | | | | |
| Lactose spray-dried | | | 42.5 | | 38.5 | 43.9 | 43.9 |
| Cellulose, microcrystalline | 30.0 | | | | 30.0 | 40.0 | 30.0 |
| Calcium hydrogen phosphate | | 30.0 | 30.0 | 68.5 | | | |
| Citric acid | | | | | | | 10.0 |
| Crospovidone | | | | 4.0 | | | |
| Croscarmellose | | | | | 4.0 | 3.0 | 3.0 |
| Magnesium stearate | 0.5 | 0.5 | 0.5 | 0.5 | 0.5 | 0.6 | 0.6 |
| Total mass (mg) | 85.0 | 85.0 | 85.0 | 85.0 | 85.0 | 100.0 | 100.0 |

Micronized rivaroxaban according to example 33 was dry-mixed with the other excipients. The resulting mixture was directly compressed to form tablets.

### Example 37

### Preparation of tablets by dry granulation

**Tablet compositions were prepared as follows:**

| | **5A** | **5B** | **5C** | **5D** | **5E** |
|---|---|---|---|---|---|
| Rivaroxaban | 10.0 | 10.0 | 10.0 | 10.0 | 10.0 |
| Sodium lauryl sulfate | 2.0 | 2.0 | 2.0 | 2.0 | 2.0 |
| Mannitol | 42.5 | 42.5 | | | |
| Lactose spray-dried | | | 42.5 | | 38.5 |
| Cellulose, microcrystalline | 30.0 | | | | 30.0 |
| Calcium hydrogen phosphate | | 30.0 | 30.0 | 68.5 | |
| Crospovidone | | | | 4.0 | |
| Croscarmellose | | | | | 4.0 |
| Magnesium stearate | 0.5 | 0.5 | 0.5 | 0.5 | 0.5 |
| Total mass (mg) | 85.0 | 85.0 | 85.0 | 85.0 | 85.0 |

Rivaroxaban was mixed with the other excipients, except Magnesium stearate, sieved and mixed again. The mixture was dry granulated with a roller-compactor using a Fitzpatrik laboratory machine. The resulting compact was milled into granules, mixed with Magnesium stearate and compressed into tablets.

### Example 38

### Preparation of tablets by hot melt granulation

**Tablet compositions were prepared as follows:**

| | **6A** | **6B** | **6C** |
|---|---|---|---|
| Rivaroxaban | 10.0 | 10.0 | 10.0 |
| PEG 6000 | 15.0 | | |
| Poloxamer 188 | | 15.0 | 15.0 |
| Mannitol | 41.5 | 43.5 | |
| Maize starch | | | 43.5 |
| Crospovidone | 8.0 | 6.0 | 6.0 |
| Cellulose, microcrystalline | 10.0 | 10.0 | 10.0 |
| Colloidal silicon dioxide | 0.5 | 0.5 | 0.5 |
| Total mass (mg) | 85.0 | 85.0 | 85.0 |

Rivaroxaban was mixed with the other excipients (except colloidal silicon dioxide) in a high-shear granulation machine at a temperature of about 55-70 °C for about 5 to 15 minutes. The mixture was cooled down to ambient temperature and mixed with colloidal silicon dioxide. The resulting mixture was compressed into tablets.

## Claims

1. Process for preparing rivaroxaban or a pharmaceutically acceptable salt or solvate thereof comprising the following steps:
a) dissolving rivaroxaban in a solvent or a mixture of solvents
b) crystallization of rivaroxaban
c) washing and drying the crystals and
d) obtaining pure rivaroxaban
wherein pure rivaroxaban has a purity at least 99,0 area % as determined by HPLC,
wherein the crystallization according to b) is achieved either by
(i) decreasing the temperature of a rivaroxaban crystallization mixture comprising rivaroxaban and a solvent from a temperature between room temperature and the reflux temperature of the solvent to a temperature within the range -20 °C to 40 °C,
(ii) the addition of an anti-solvent to a solution of rivaroxaban in a solvent, or
(iii) the addition of a solution of rivaroxaban in a solvent to an anti-solvent,
the solvent from step (i) being selected from 2-butanone, 3-pentanone, acetonitrile, 1-butanol and mixtures thereof, and
the solvent from steps (ii) and (iii) being selected from DMSO, DMF, DMA, acetic acid or mixtures thereof.

2. The process according to claim 1, **characterized in that** the anti-solvent from steps (ii) and (iii) is selected from water, alcohols such as methanol, ethanol, isopropanol, butanol; esters such as ethyl acetate, isopropyl acetate; ethers such as THF, dioxane, tert-butyl methyl ether, diisopropyl ether; acetonitrile, alkanes, halogenated alkanes, aromatic carbonhydrates, and mixtures thereof.

3. The process of any of claims 1 to 2, **characterized in that** the pure rivaroxaban has purity at least 99,5 area % as determined by HPLC.

4. The process of any of claims 1 to 3, **characterized in that** pure rivaroxaban has purity at least 99,7 area % as determined by HPLC.

5. The process of any of the preceding claims wherein a pure rivaroxaban is in a crystal form I.

6. The process of any of the preceding claims wherein a pure rivaroxaban is stable for at least 18 months when stored at 25 °C at 60 % relative humidity (RH).

7. The process of any of the preceding claims wherein obtained product contains less than 0,2 area % of des-chloro rivaroxaban as determined by HPLC.

8. The process of any of the preceding claims wherein obtained product contains less than 0,1 area % of des-chloro rivaroxaban as determined by HPLC.

9. The process of any of the preceding claims wherein obtained product contains less than 0,05 area % of des-chloro rivaroxaban as determined by HPLC.

10. The process according to any of the preceding claims **characterized in that** the rivaroxaban is synthesized by a method of synthesizing a rivaroxaban that comprises an amount of des-chloro rivaroxaban selected from not greater than about
A) 0,2 %,
B) not greater than about 0,15 %, or
C) not greater than about 0,10 %
which method comprises:
a) obtaining one or more samples of one or more compound of formula (II) batches; 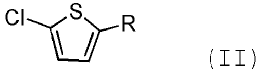
b) measuring the level of compound of formula (III) 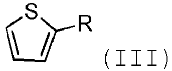 in each of the samples of (a);
c) selecting a compound of formula (II) batch that comprises a purity of compound of formula (II) based on the measurement of compound of formula (III) conducted in (b); and
d) using the batch selected in (c) to synthesize rivaroxaban,
wherein R of formulas (II) and (III) are defined with R being - COOH, COX, wherein X is halogen selected from Cl, F, Br, J, or compounds (II) and (III) are anhydrides.

## Patentansprüche

1. Verfahren zum Herstellen von Rivaroxaban oder einem pharmazeutisch verträglichen Salz oder Solvat davon, umfassend die folgenden Schritte:
a) Lösen von Rivaroxaban in einem Lösungsmittel oder einem Gemisch von Lösungsmitteln
b) Kristallisation von Rivaroxaban
c) Waschen und Trocknen der Kristalle und
d) Erhalten von reinem Rivaroxaban,
wobei reines Rivaroxaban eine Reinheit von mindestens 99,0 Flächen-% wie durch HPLC bestimmt aufweist,
wobei die Kristallisation gemäß b) erreicht wird entweder durch
(i) Erniedrigen der Temperatur eines Rivaroxaban-Kristallisationsgemisches umfassend Rivaroxaban und ein Lösungsmittel von einer Temperatur zwischen Raumtemperatur und der Rückflusstemperatur des Lösungsmittels auf eine Temperatur im Bereich von -20 °C bis 40 °C,
(ii) die Zugabe eines Antilösungsmittels zu einer Lösung von Rivaroxaban in einem Lösungsmittel, oder
(iii) die Zugabe einer Lösung von Rivaroxaban in einem Lösungsmittel zu einem Antilösungsmittel,
wobei das Lösungsmittel von Schritt (i) aus 2-Butanon, 3-Pentanon, Acetonitril, 1-Butanol und Gemischen davon ausgewählt ist, und
das Lösungsmittel von Schritten (ii) und (iii) aus DMSO, DMF, DMA, Essigsäure oder Gemischen davon ausgewählt ist.

2. Verfahren gemäß Anspruch 1, **dadurch gekennzeichnet, dass** das Antilösungsmittel von Schritten (ii) und (iii) aus Wasser, Alkoholen wie Methanol, Ethanol, Isopropanol, Butanol; Estern wie Ethylacetat, Isopropylacetat; Ethern wie THF, Dioxan, *tert-*Butylmethylether, Diisopropylether; Acetonitril, Alkanen, halogenierten Alkanen, aromatischen Kohlenhydraten, und Gemischen davon ausgewählt ist.

3. Verfahren nach einem der Ansprüche 1 bis 2, **dadurch gekennzeichnet, dass** das reine Rivaroxaban eine Reinheit von mindestens 99,5 Flächen-% wie durch HPLC bestimmt aufweist.

4. Verfahren nach einem der Ansprüche 1 bis 3, **dadurch gekennzeichnet, dass** reines Rivaroxaban eine Reinheit von mindestens 99,7 Flächen-% wie durch HPLC bestimmt aufweist.

5. Verfahren nach einem der vorhergehenden Ansprüche, wobei ein reines Rivaroxaban in einer Kristallform I vorliegt.

6. Verfahren nach einem der vorhergehenden Ansprüche, wobei ein reines Rivaroxaban mindestens 18 Monate lang stabil ist, wenn es bei 25 °C bei 60 % relativer Feuchtigkeit (RH) gelagert wird.

7. Verfahren nach einem der vorhergehenden Ansprüche, wobei erhaltenes Produkt weniger als 0,2 Flächen-% Deschlororivaroxaban wie durch HPLC bestimmt enthält.

8. Verfahren nach einem der vorhergehenden Ansprüche, wobei erhaltenes Produkt weniger als 0,1 Flächen-% Deschlororivaroxaban wie durch HPLC bestimmt enthält.

9. Verfahren nach einem der vorhergehenden Ansprüche, wobei erhaltenes Produkt weniger als 0,05 Flächen-% Deschlororivaroxaban wie durch HPLC bestimmt enthält.

10. Verfahren gemäß einem der vorhergehenden Ansprüche, **dadurch gekennzeichnet, dass** das Rivaroxaban synthetisiert wird durch ein Verfahren von Synthetisieren eines Rivaroxabans, welches eine Menge an Deschlororivaroxaban umfasst, die ausgewählt ist von nicht höher als etwa
A) 0,2 %,
B) nicht höher als etwa 0,15 %, oder
C) nicht höher als etwa 0,10 %,
wobei das Verfahren umfasst:
a) Erhalten von einer oder mehr Proben von einer oder mehr Verbindung von Formel (II)-Chargen; 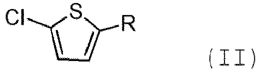
b) Messen der Menge von Verbindung von Formel (III) 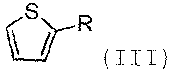 in jeder der Proben von (a);
c) Auswählen einer Verbindung von Formel (II)-Charge, die eine Reinheit von Verbindung von Formel (II) umfasst, basierend auf der in (b) durchgeführten Messung von Verbindung von Formel (III); und
d) Verwenden der in (c) ausgewählten Charge zum Synthetisieren von Rivaroxaban, wobei R von Formeln (II) und (III) mit R, welches -COOH, COX ist, definiert sind, wobei X aus Cl, F, Br, J ausgewähltes Halogen ist, oder Verbindungen (II) und (III) Anhydride sind.

## Revendications

1. Procédé pour préparer du rivaroxaban ou un sel ou solvate pharmaceutiquement acceptable de celui-ci, comprenant les étapes suivantes :
a) dissolution de rivaroxaban dans un solvant ou un mélange de solvants
b) cristallisation du rivaroxaban
c) lavage et séchage des cristaux et
d) obtention de rivaroxaban pur
dans lequel le rivaroxaban pur a une pureté d'au moins 99,0 % en surface telle que déterminée par CLHP,
dans lequel la cristallisation conformément à b) est obtenue soit par
(i) abaissement de la température d'un mélange de cristallisation de rivaroxaban comprenant du rivaroxaban et un solvant, d'une température comprise entre la température ambiante et la température de reflux du solvant, à une température située dans la plage allant de -20°C à 40°C,
(ii) addition d'un anti-solvant à une solution de rivaroxaban dans un solvant, ou
(iii) addition d'une solution de rivaroxaban dans un solvant à un anti-solvant,
le solvant de l'étape (i) étant choisi parmi la 2-butanone, la 3-pentanone, l'acétonitrile, le 1-butanol et leurs mélanges, et
le solvant des étapes (ii) et (iii) étant choisi parmi le DMSO, le DMF, le DMA, l'acide acétique et leurs mélanges.

2. Procédé selon la revendication 1, **caractérisé en ce que** l'anti-solvant des étapes (ii) et (iii) est choisi parmi l'eau, les alcools tels que le méthanol, l'éthanol, l'isopropanol, le butanol ; les esters tels que l'acétate d'éthyle, l'acétate d'isopropyle ; les éthers tels que le THF, le dioxane, le tert-butyl-méthyléther, le diisopropyléther ; l'acétonitrile, les alcanes, les alcanes halogénés, les hydrates de carbone aromatiques, et leurs mélanges.

3. Procédé selon l'une quelconque des revendications 1 et 2, **caractérisé en ce que** le rivaroxaban pur a une pureté d'au moins 99,5 % en surface, telle que déterminée par CLHP.

4. Procédé selon l'une quelconque des revendications 1 à 3, **caractérisé en ce que** le rivaroxaban pur a une pureté d'au moins 99,7 % en surface, telle que déterminée par CLHP.

5. Procédé selon l'une quelconque des revendications précédentes, dans lequel le rivaroxaban pur est sous la forme cristalline I.

6. Procédé selon l'une quelconque des revendications précédentes, dans lequel le rivaroxaban pur est stable pendant au moins 18 mois quand il est stocké à 25°C sous une humidité relative (HR) de 60 %.

7. Procédé selon l'une quelconque des revendications précédentes, dans lequel le produit obtenu contient moins de 0,2 % en surface de des-chlororivaroxaban, tel que déterminé par CLHP.

8. Procédé selon l'une quelconque des revendications précédentes, dans lequel le produit obtenu contient moins de 0,1 % en surface de des-chlororivaroxaban, tel que déterminé par CLHP.

9. Procédé selon l'une quelconque des revendications précédentes, dans lequel le produit obtenu contient moins de 0,05 % en surface de des-chlororivaroxaban, tel que déterminé par CLHP.

10. Procédé selon l'une quelconque des revendications précédentes, **caractérisé en ce que** le rivaroxaban est synthétisé par une méthode de synthèse de rivaroxaban qui comprend une quantité de des-chlororivaroxaban choisie parmi pas plus d'environ
A) 0,2 %,
B) pas plus d'environ 0,15 %, ou
C) pas plus d'environ 0,10 %
laquelle méthode comprend :
a) l'obtention d'un ou plusieurs échantillons d'un ou plusieurs lots de composé de formule (II) ; 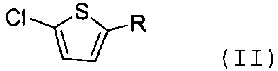
b) la mesure du niveau de composé de formule (III) 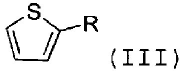 dans chacun des échantillons de (a) ;
c) la sélection d'un lot de composé de formule (II) qui comprend une pureté du composé de formule (II) basée sur la mesure du composé de formule (III) effectuée en (b) ; et
d) l'utilisation du lot sélectionné en (c) pour synthétiser du rivaroxaban,
dans lequel R dans les formules (II) et (III) est défini comme R étant -COOH, COX, où X est un halogène choisi parmi Cl, F, Br, J, ou les composés (II) et (III) sont des anhydrides.
